# EUROPEAN PATENT SPECIFICATION

(11) **EP 0 637 248 B1**
(45) Date of publication and mention of the grant of the patent: **03.07.2002**
(21) Application number: 93911768.5
(22) Date of filing: 20.04.1993
(51) Int. Cl.: A61K 38/00, A61K 9/107

(54) **PHARMACEUTICAL COMPOSITION CONTAINING CYCLOSPORIN DERIVATIVE**
ARZNEIMITTEL ENTHALTENDE CYCLOSPORINDERIVATE
COMPOSITION PHARMACEUTIQUE CONTENANT UN DERIVE DE CYCLOSPORINE

(30) Priority: 22.04.1992 GB 9208712
(43) Date of publication of application: 08.02.1995
(73) Proprietor: Novartis AG, 4056 Basel (CH); Novartis-Erfindungen Verwaltungsgesellschaft m.b.H., 1230 Wien (AT)
(72) Inventor: MEINZER, Armin, D-7800 Freiburg/Munzingen (DE); RICHTER, Friedrich, CH-3322 Schönbühl-Urtenen (CH); VONDERSCHER, Jacky, Francis, F-68400 Riedisheim (FR)
(86) International application number: EP9300955
(87) International publication number: WO9320833

(56) References cited:
- EP-A- 0 296 122
- GB-A- 2 015 339
- GB-A- 2 222 770

## Description

This invention relates to a pharmaceutical formulation having as the active ingredient a cyclosporin derivative that is useful in the treatment of multiple drug resistance syndrome. In particular this invention relates to a pharmaceutical formulation that contains ([3'-desoxy-3'-oxo-MeBmt]¹-[Val]²-Ciclosporin) as an active ingredient.

The resistance of tumour cells to chemotherapeutics is a major cause in the failure of chemotherapy. One form of resistance to chemotherapeutics is "multiple drug resistance" where the tumour cells become cross-resistant to a variety of chemotherapeutics; for example alkaloids, anthracyclines, actinomyxin D, adriamycin and colchicine. Multiple drug resistance syndrome has been correlated in many reports with the overexpression of the transmembrane glycoproteins called P-glycoproteins (Pgp). Cyclosporins have been found to reverse multiple drug resistance syndrome and [3'-desoxy-3'-oxo-MeBmt]¹-[Val]²-Ciclosporin in particular has been found to be effective. [3'-desoxy-3'-oxo-MeBmt]¹-[Val]²-Ciclosporin and its utility is described in detail in European patent publication No 296 122.

The cyclosporins comprise a class of structurally distinctive, cyclic, poly-N-methylated undecapeptides, generally possessing pharmacological, in particular immunosuppressive, anti-inflammatory and/or anti-parasitic activity, each to a greater or lesser degree. Cyclosporins are generally very insoluble in aqueous media. Consequently, difficulties have arisen in developing pharmaceutically acceptable carriers which allow delivery of the drug in sufficiently high concentrations to permit convenient use and which allow efficient and consistent absorption of the drug by the body. Also specific cyclosporins present very specific problems in relation to their administration and, in particular, in providing galenic formulations. Species specific problems also arise with regard to drug bioavailability and variability in patient dose response. This also applies to the compound ([3'-desoxy-3'-oxo- MeBmt]¹-[Val]²-Ciclosporin) which presents very specific problems in relation to providing galenic formulations containing it.

To deal with the general problems of formulating cyclosporins, British patent application 2 222 770 A discloses galenic formulations, containing cyclosporins, that are in the form of microemulsions or microemulsion preconcentrates. These formulations comprise a hydrophilic phase, a lipophilic phase and a surfactant. The following components for the hydrophilic phase are given: Transcutol, Glycofurol and 1,2-propylene glycol. Medium chain fatty acid triglycerides are disclosed as being suitable for the lipophilic phase. Reaction products of natural or hydrogenated vegetable oils and ethylene glycol are given as surfactants. However this British patent application did not deal with the problems of formulating ([3'-desoxy-3'-oxo-MeBmt]¹-[Val]²- Ciclosporin) which is much more lipophilic than most other cyclosporins.

It has now been surprisingly found that stable formulations containing ([3'-desoxy-3'-oxo-MeBmt]¹-[Val]²-Ciclosporin) that have good bioavailability and good inter-subject variability characteristics can be obtained.

Accordingly this invention provides a pharmaceutical composition that comprises ([3'-desoxy-3'-oxo- MeBmt]¹-[Val]²-Ciclosporin) as an active ingredient and a carrier medium comprising (1) a hydrophilic phase; (2) a transesterified ethoxylated vegetable oil; and (3) a surfactant. Preferably the formulation is in the form of a microemulsion or microemulsion preconcentrate.

This composition provides a surprisingly high bioavailability (about twice that of a conventional Ciclosporin formulation) and hence the dosage of ([3'-desoxy-3'-oxo- MeBmt]¹-[Val]²-Ciclosporin) required to be delivered can be decreased. The composition is also convenient to use and permits efficient and consistent absorption of ([3'-desoxy-3'-oxo- MeBmt]¹-[Val]²-Ciclosporin) by the body. The composition is particularly effective in treating multiple drug resistance syndrome in, for example, cancer patients undergoing chemotherapy.

Preferably the composition is in the form of a "microemulsion preconcentrate", in particular of the type providing o/w (oil-in-water) microemulsions. However the composition may be in the form of a microemulsion which additionally contains an aqueous phase; preferably water.

A "microemulsion preconcentrate" is defined in this specification as being a formulation which spontaneously forms a microemulsion in an aqueous medium, for example, in water or in the gastric juices after oral application. The formation of such a microemulsion is highly desirable to overcome the problems discussed above.

A "microemulsion" is a non-opaque or substantially non-opaque colloidal dispersion that is formed spontaneously or substantially spontaneously when its components are brought into contact. A microemulsion is thermodynamically stable and contains dispersed particles of a size less than about 2000 Å (20nm). Generally microemulsions comprise droplets or particles having a diameter of less than about 1500 Å; (15nm) typically from 100 to 1000 Å 1 to 10 nm. Further characteristic can be found in British patent application 2 222 770 A.

A "pharmaceutical composition" means a composition in which the individual components or ingredients are themselves pharmaceutically acceptable and, when a particular form of administration is foreseen, are suitable or acceptable for that form of administration.

The transesterified ethoxylated vegetable oil may comprise 10 to 85 % by weight of the carrier medium; preferably 15 to 70 % by weight, more preferably 20 to 60 % by weight and even more preferably about 20 % by weight.

The surfactant may comprise 5 to 80 % by weight of the carrier medium; preferably 10 to 70 % by weight, more preferably 20 to 60 % by weight and even more preferably about 60 % by weight.

The hydrophilic phase may comprise 10 to 50 % by weight of the carrier medium; preferably 15 to 40 % by weight, more preferably 20 to 30 % by weight and even more preferably about 20 % by weight.

The hydrophilic phase may be selected from Transcutol® (which has the formula C₂H₅-[O-(CH₂)₂]₂-OH), Glycofurol® (also known as tetrahydrofurfuryl alcohol polyethylene glycol ether) and 1,2-propylene glycol, or mixtures thereof, and is preferably 1,2-propylene glycol. The hydrophilic phase may include further hydrophilic co-components, for example ethanol. These co-components will generally be present in partial replacement of other components of the hydrophilic phase. While the use of ethanol in the compositions is not essential, it has been found to be of particular advantage when the compositions are to be manufactured in soft gelatine, encapsulated form. This is because storage characteristics are improved, in particular the risk of cyclosporin precipitation following encapsulation procedures is reduced. Thus the shelf life stability may be extended by employing ethanol or some other such co-component as an additional ingredient of the hydrophilic phase. The ethanol may comprises 0 to 60 % by weight of the hydrophilic phase; preferably 20 to 55% by weight and more preferably about 55 % by weight.

The active ingredient is preferably present in an amount of 5 to 15 % by weight of the composition; more preferably about 10 %.

Suitable transesterified ethoxylated vegetable oils may be obtained by reacting various natural vegetable oils (for example, maize oil, kernel oil, almond oil, ground nut oil, olive oil, soybean oil, sunflower oil, safflower oil and palm oil, or mixtures thereof) with polyethylene glycols that have an average molecular weight of from 200 to 800, in the presence of an appropriate catalyst. These procedures are known and an example is described in US Patent 3 288 824. Transesterified ethoxylated corn oil is particularly preferred.

Transesterified ethoxylated vegetable oils are known and are commercially available under the trade name Labrafil (see H. Fiedler, Lexikon der Hilfsstoffe, 3^{rd} edition, vol 2, page 707). Examples are Labrafil® M 2125 CS (obtained from corn oil and having an acid number of less than about 2, a saponification number of 155 to 175, an HLB value of 3 to 4, and an iodine number of 90 to 110), and Labrafil M 1944 CS (obtained from kernel oil and having an acid number of about 2, a saponification number of 145 to 175 and an iodine number of 60 to 90). Labrafil M 2130 CS (which is a transesterification product of a C₁₂₋₁₈ glyceride and polyethylene glycol and which has a melting point of about 35 to 40°C, an acid number of less than about 2, a saponification number of 185 to 200 and an iodine number of less than about 3) may also be used. The preferred transesterified ethoxylated vegetable oil is Labrafil® M 2125 CS which can be obtained, for example, from Gattefossé, Saint-Priest Cedex, France.

The surfactant may comprise reaction products of a natural or hydrogenated castor oil and ethylene oxide. The natural or hydrogenated castor oil may be reacted with ethylene oxide in a molar ratio of from about 1:35 to about 1:60, with optional removal of the polyethyleneglycol component from the products. Various such surfactants are commercially available. The polyethyleneglycol-hydrogenated castor oils available under the trade name Cremophor are especially suitable. Particularly suitable are Cremophor® RH 40, which has a saponification number of about 50 to 60, an acid number less than about 1, a water content ( Fischer) less than about 2%, an n_{D}⁶⁰ of about 1.453 to 1.457 and an HLB of about 14 to 16; Cremophor RH 60, which has a saponification number of about 40 to 50, an acid number less than about 1, an iodine number of less than about 1, a water content (Fischer) of about 4.5 to 5.5%, an n_{D}²⁵ of about 1.453 to 1.457 and an HLB of about 15 to 17; and Cremophor® EL, which has a molecular weight (by steam osmometry) of about 1630, a saponification number of about 65 to 70, an acid number of about 2, an iodine number of about 28 to 32 and an n_{D}²⁵ of about 1.471.

Similar or identical products which may also be used are available under the trade names Nikkol (e.g. Nikkol HCO-40 and HCO-60), Mapeg (e.g. Mapeg CO-40h), Incrocas (e.g. Incrocas 40), and Tagat (e.g. Tagat RH 40). These surfactants are further described in Fiedler loc. cit..

The surfactant may also be selected from polyoxyethylene sorbitan fatty acid esters, such as mono- and tri-lauryl, palmityl, stearyl and oleyl esters. Examples of commercially available esters are those available under the trade name Tween (see Fiedler, pages 1300 to 1304) and particularly Tween 60® (polyoxyethylene(20) sorbitan mono stearate) and Tween 80 (polyoxyethylene(20) sorbitan mono oleate).

Preferably the surfactant is Cremophor® RH 40.

The compositions may also include further additives or ingredients, for example antioxidants (such as ascorbyl palmitate, butyl hydroxy anisole (BHA), butyl hydroxy toluene (BHT) and tocopherols) and/or preserving agents. These additives or ingredients may comprise about 0.05 to 1% by weight of the total weight of the composition. The compositions may also include sweetening or flavoring agents in an amount of up to about 2.5 or 5% by weight based on the total weight of the composition. Preferably the antioxidant is α-tocopherol (vitamin E).

The compositions exhibit especially advantageous properties when administered orally; for example in terms of consistency and high level of bioavailability obtained in standard bioavailability trials. These trials are performed in healthy patients using a nonspecific monoclonal kit to determine the level of ([3'-desoxy-3'-oxo-MeBmt]¹-[Val]²-Ciclosporin). Results have shown that at least 170% greater bioavailability can be obtained in mammals and about 200% in humans (as compared to the commercially available, oral Ciclosporin formulation). This means that the dosage to be delivered can be reduced as compared to the commercial oral form. The compositions also provide less food interaction, which has been observed with the commercially available oral form of Ciclosporin; especially with fat rich food. Moreover, inter-subject and intra-subject variability of pharmacokinetic parameters may be significantly lower with the compositions than with the commercial oral form of Ciclosporin. Specifically the difference between the pharmacokinetic parameters with food intake and without food intake, or even between day time absorption and night time absorption, may be reduced.

Thus pharmacokinetic parameters, for example absorption and blood levels, become surprisingly more predictable and problems in administration with erratic absorption may be eliminated or reduced. Additionally the composition is compatible with tenside materials, for example bile salts, present in the gastro-intestinal tract. That is, the composition is fully dispersible in aqueous systems comprising such natural tensides and is thus capable of providing microemulsion systems in situ which are stable and do not exhibit precipitation of the active ingredient or other disruption of fine particulate structure. The function of the composition upon oral administration remains substantially independent of and/or unimpaired by the relative presence or absence of bile salts at any particular time or for any given individual.

The compositions are preferably compounded in unit dosage form, for example by filling them into orally administrable capsule shells. The capsule shells may be soft or hard gelatine capsule shells. Where compositions are in unit dosage form, each unit dosage will suitably contain between 10 and 200 mg [3'-desoxy-3'-oxo-MeBmt]¹-[Val]²- Ciclosporin, more preferably between 10 and 150 mg; for example 15, 20, 25, 50 or 100 mg [3'-desoxy-3'-oxo-MeBmt]¹-[Val]²-Ciclosporin. Such unit dosage forms are suitable for administration 1 to 5 times daily depending upon the particular purpose of therapy, the phase of therapy and the like.

However, if desired, the composition may be in drink solution form and may include water or any other aqueous system, to provide microemulsion systems suitable for drinking.

The utility of the compositions can be observed in standard clinical tests such as those disclosed in EP 296 122 using dosages in the range of 200 mg to 1800 mg per day for a 75 kilogram adult and in standard animal models. The increased bioavailability of the active ingredient provided by the compositions can be observed in standard animal tests such as described in example 13 and in clinical trials.

The optimal dosage to be administered to a particular patient must be considered carefully by the treating physician as individual response to and metabolism of [3'-desoxy-3'-oxo-MeBmt]¹-[Val]²-Ciclosporin may vary. It may be advisable to monitor the blood serum levels of the cyclosporin by radioimmunoassay, monoclonal antibody assay, or other appropriate conventional means. Dosages of [3'-desoxy-3'-oxo-MeBmt]¹- [Val]²-Ciclosporin for treatment of multiple drug resistance syndrome will generally range from 200 mg to 1800 mg per day for a 75 kilogram adult, preferably 300 mg to 1500 mg, with the optimal dosage being approximately 500 mg per day. Satisfactory results are obtained by administering about 500 mg per day in the form of five capsules each containing 100 mg or ten capsules each containing 50 mg.

In a further aspect the invention also provides a process for the production of a pharmaceutical composition as defined above, which process comprises bringing (1) a hydrophilic phase; (2) a transesterified ethoxylated vegetable oil; and (3) a surfactant into intimate admixture in relative proportions of components (1), (2) and (3) and adding the active ingredient. When required the composition may be compounded into unit dosage form, for example filing the composition into gelatine capsules.

Optionally further components or additives, in particular a hydrophilic phase co-component, for example ethanol, may be mixed with components (1), (2) and (3) or with or after addition of the active ingredient.

The composition may be combined with sufficient water or sufficient of an aqueous solvent medium such that a microemulsion is obtained.

Embodiments of the invention are now described, by way of example only, with reference to the drawings in which:

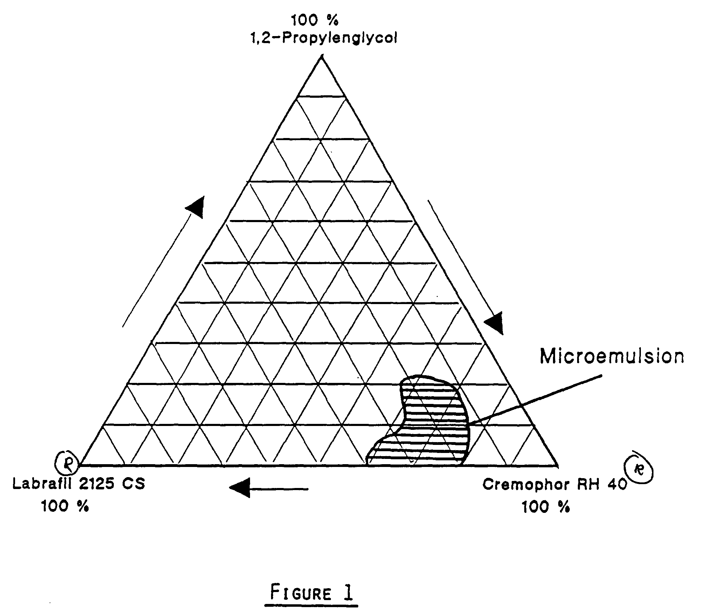

Figure 1 shows a three way plot of the range of concentrations of preferred components of the carrier medium which result in microemulsion preconcentrates with the concentration of the active ingredient remaining constant at 10% and that of ethanol at 15%. Formulations within the shaded area form microemulsions spontaneously in aqueous media. Formulations outside of the shaded area form other disperse systems, e.g., emulsions, in aqueous media.

The following examples illustrate some preferred forms of the invention.

**Table 1:**

| Examples 1 to 4 | | | | |
|---|---|---|---|---|
| Ingredients/Example | 1 | 2 | 3 | 4 |
| Active Ingredient | 100 mg | 100 mg | 100 mg | 100 mg |
| Ethanol absolute | 105 mg | 150 mg | 100 mg | 150 mg |
| Labrafil® M 2125 CS | 150 mg | 150 mg | 640 mg | 150 mg |
| 1,2-propylene glycol | 95 mg | 75 mg | 80 mg | 150 mg |
| Cremophor® RH 40 | 525 mg | 525 mg | 80 mg | 450 mg |
| α-tocopherol | 1 mg | 1 mg | 1 mg | 1 mg |
| Tween 80® | - | - | - | - |
| Total | 1001 mg | 1001 mg | 1001 mg | 1001 mg |

**Table 2:**

| Examples 5 to 8 | | | | |
|---|---|---|---|---|
| Ingredients/Example | 5 | 6 | 7 | 8 |
| Active Ingredient | 100 mg | 100 mg | 100 mg | 100 mg |
| Ethanol absolute | 100 mg | 100 mg | 100 mg | 100 mg |
| Labrafil® M 2125 CS | 160 mg | 240 mg | 160 mg | 320 mg |
| 1,2-propylene glycol | 80 mg | 80 mg | 160 mg | 80 mg |
| Cremophor® RH 40 | 560 mg | 480 mg | 480 mg | 400 mg |
| α-tocopherol | 1 mg | 1 mg | 1 mg | 1 mg |
| Tween 80® | - | - | - | - |
| Total | 1001 mg | 1001 mg | 1001 mg | 1001 mg |

**Table 3:**

| Examples 9 to 12 | | | | |
|---|---|---|---|---|
| Ingredients/Example | 9 | 10 | 11 | 12 |
| Active Ingredient | 100 mg | 100 mg | 100 mg | 100 mg |
| Ethanol absolute | 100 mg | 100 mg | 100 mg | 100 mg |
| Labrafil® M 2125 CS | 160 mg | 240 mg | 480 mg | 560 mg |
| 1,2-propylene glycol | 160 mg | 160 mg | 160 mg | 80 mg |
| Cremophor® RH 40 | - | - | - | - |
| α-tocopherol | 1 mg | 1 mg | 1 mg | 1 mg |
| Tween 80® | 480 mg | 400 mg | 160 mg | 80 mg |
| Total | 1001 mg | 1001 mg | 1001 mg | 1001 mg |

To produce the compositions, the Labrafil® M 2125 CS, the Cremophor® RH 40 (or Tween®) and the polypropylene glycol are mixed thoroughly and then deaerated. The [3'-desoxy-3'-oxo-MeBmt]¹- [Val]²-Ciclosporin is then suspended in the mixture and the mixture again deaerated. The ethanol is then added to the mixture. It may be necessary to warm the PEG-hydrogenated castor oil slightly (e.g. to about 30°C) until it melts to a clear liquid. The composition obtained is suitable for oral administration as a drink solution or in a soft gelatin capsule containing a measured quantity, for example 1 ml, of the composition. The compositions are filled into the capsules and sealed using, for example, the Quali-Seal technique.

### Stability

The capsules are packaged in doubled sided aluminium blisters and stored at 5°C, 25°C (at 50% humidity) and 30°C (at 65 % humidity). The capsules are examined after 6 months and 12 months. No change in the appearance of the contents and the capsule shells is observed. The capsules are therefore very stable.

### EXAMPLE 13: Bioavailability in dogs

The biopharmaceutical properties of the composition of Example 1 is compared with the marketed soft-gelatine capsule of Ciclosporin. The forms are compared after oral administration to 7 male beagle dogs in a cross-over design. The pharmacokinetic profile of the active ingredient was determined in whole blood over 24 hours. The areas under the curve of the blood concentration versus time curves (AUC), Cₘₐₓ and Tₘₐₓ are determined. Doses of 50 mg or 350 mg of active ingredient are used; 2 dogs for 50 mg and 5 dogs for 350 mg.

Composition X (commercial form, soft gelatin capsule) composition:

| | |
|---|---|
| Ciclosporin | 11 % by weight |
| Labrafil® | 32 % by weight |
| ethanol | 11 % by weight |
| Maize oil | 46 % by weight |

### Drug administration:

7 male beagle dogs weighing around 12 kg are used in the trial. Twenty hours before drug administration, food is withdrawn but the animals are allowed free access to water until the beginning of the experiment. The dosage forms are administered by gavage to the animals, early in the morning (approx. 8.00 am), followed by 20 ml NaCl 0.9% solution. Three hours after the administration, the animals are again allowed free access to water and food. A wash-out period of a week is necessary between 2 administrations to the same animal.

### Blood sampling:

Blood samples of 2 ml (or 5 ml for the blank sample) are taken from the vena cephalica (forearm) using a sterile needle (diameter about 1.2 mm) and collected into 5 ml plastic tubes containing EDTA. The samples are taken before administration of the drug and at 15 minutes, 30 minutes, 45 minutes, 1 hour, 1.5 hours, 2 hours, 3 hours, 4 hours, 6 hours, 8 hours, 12 hours and 24 hours after the oral administration of the drug. The blood samples are stored at about -20°C until the drug assay is carried out. The blood samples are analysed by nonspecific radioimmunoassay (RA). The areas under the blood drug concentration versus time curves (AUC) are calculated using the trapezoidal rule. An analysis (CV) of variance is performed and the mean AUCs, Cmax and Tmax are compared statistically by the Tukey test. The results obtained are shown in the following table.

**Table 4:**

| AUC, Cmax and Tmax results in dog | | | | |
|---|---|---|---|---|
| Example | Dose (mg) | AUC (0 - 24hr) | Cmax (ng/ml) | Tmax (hrs) |
| X | 350 | 10851 | 1299 | 2.00 |
| X | 50 | 1926 | 477 | 1.25 |
| 1 | 350 | 18040 | 2092 | 1.80 |
| 1 | 50 | 4139 | 602 | 1.25 |

Conclusion: The composition of Example 1 has a significantly higher bioavailability (factor 1.7 at a dosage of 350 mg and factor 2.1 at a dosage of 50 mg) than the commercial, soft-gelatin capsule of Ciclosporin.

## Claims

1. A pharmaceutical composition that comprises ([3'-desoxy-3'-oxo-MeBmt]¹-[Val]²-Ciclosporin) as an active ingredient and a carrier medium comprising
1) a hydrophilic phase;
2) a transesterified ethoxylated vegetable oil; and
3) a surfactant;
which composition is in the form of a microemulsion preconcentrate of the type providing o/w (oil-in-water) microemulsions.

2. A pharmaceutical composition according to claim 1 in which the transesterified ethoxylated vegetable oil comprises 10 to 85 % by weight of the carrier medium; the surfactant comprises 5 to 80 % by weight of the carrier medium; and the hydrophilic phase comprises 10 to 50 % by weight of the carrier medium.

3. A pharmaceutical composition according to claim 2 in which the transesterified ethoxylated vegetable oil comprises 15 to 70 % by weight of the carrier medium; the surfactant comprises 10 to 70 % by weight of the carrier medium; and the hydrophilic phase comprises 15 to 40 % by weight of the carrier medium.

4. A pharmaceutical composition according to claim 3 in which the transesterified ethoxylated vegetable oil comprises about 20 % by weight of the carrier medium; the surfactant comprises about 60 % by weight of the carrier medium; and the hydrophilic phase comprises about 20 % by weight of the carrier medium.

5. A pharmaceutical composition according to any preceding claim in which the hydrophilic phase includes a component selected from Transcutol, Glycofurol and 1,2-propylene glycol, or mixtures thereof.

6. A pharmaceutical composition according to claim 5 in which the hydrophilic phase includes ethanol as a further hydrophilic co-component.

7. A pharmaceutical composition according to claim 5 in which the ethanol comprises 0 to 60 % by weight of the hydrophilic phase.

8. A pharmaceutical composition according to any preceding claim in which the active ingredient is present in an amount of 5 to 15 % by weight of the composition.

9. A pharmaceutical composition according to any preceding claim in which the transesterified ethoxylated vegetable oil is a transesterified ethoxylated corn oil.

10. A pharmaceutical composition according to any preceding claim in which the surfactant is a reaction product of a natural or hydrogenated castor oil and ethylene oxide, or is a polyoxyethylene sorbitan fatty acid ester.

11. A pharmaceutical composition according to any preceding claim for use in the treatment of multiple drug resistance syndrome in humans.

12. A process for the production of a pharmaceutical composition as defined in claim 1, which process comprises mixing together proportions of the hydrophilic phase, the transesterified ethoxylated vegetable oil, and the surfactant and adding the active ingredient.

## Patentansprüche

1. Pharmazeutische Zusammensetzung, die [3'-Desoxy-3'-oxo-MeBmt]¹-[Val]²-Ciclosporin als Wirkstoff und ein Trägermedium enthält, das umfaßt
1) eine hydrophile Phase
2) ein umgeestertes ethoxyliertes Pflanzenöl, und
3) ein oberflächenaktives Mittel,
wobei die Zusammensetzung in Form eines Mikroemulsionspräkonzentrats des Typs vorliegt, der O/W (Öl in Wasser) Mikroemulsionen bereitstellt.

2. Pharmazeutische Zusammensetzung nach Anspruch 1, worin das umgeesterte ethoxylierte Pflanzenöl 10 bis 85 Gewichtsprozent des Trägermediums umfaßt, das oberflächenaktive Mittel 5 bis 80 Gewichtsprozent des Trägermediums umfaßt und die hydrophile Phase 10 bis 50 Gewichtsprozent des Trägermediums umfaßt.

3. Pharmazeutische Zusammensetzung nach Anspruch 2, worin das umgeesterte ethoxylierte Pflanzenöl 15 bis 70 Gewichtsprozent des Trägermediums umfaßt, das oberflächenaktive Mittel 10 bis 70 Gewichtsprozent des Trägermediums umfaßt und die hydrophile Phase 15 bis 40 Gewichtsprozent des Trägermediums umfaßt.

4. Pharmazeutische Zusammensetzung nach Anspruch 3, worin das umgeesterte ethoxylierte Pflanzenöl etwa 20 Gewichtsprozent des Trägermediums umfaßt, das oberflächenaktive Mittel etwa 60 Gewichtsprozent des Trägermediums umfaßt und die hydrophile Phase etwa 20 Gewichtsprozent des Trägermediums umfaßt.

5. Pharmazeutische Zusammensetzung nach einem der vorangehenden Ansprüche, worin die hydrophile Phase eine Komponente umfaßt, die ausgewählt ist aus Transcutol, Glycofurol und 1,2-Propylenglycol oder Gemischen hiervon.

6. Pharmazeutische Zusammensetzung nach Anspruch 5, worin die hydrophile Phase Ethanol als weitere hydrophile Co-Komponente enthält.

7. Pharmazeutische Zusammensetzung nach Anspruch 5, worin das Ethanol 0 bis 60 Gewichtsprozent der hydrophilen Phase umfaßt.

8. Pharmazeutische Zusammensetzung nach einem der vorangehenden Ansprüche, worin der Wirkstoff in einer Menge von 5 bis 15 Gewichtsprozent der Zusammensetzung vorhanden ist.

9. Pharmazeutische Zusammensetzung nach einem der vorangehenden Ansprüche, worin das umgeesterte ethoxylierte Pflanzenöl ein umgeestertes ethoxyliertes Maisöl ist.

10. Pharmazeutische Zusammensetzung nach einem der vorangehenden Ansprüche, worin das oberflächenaktive Mittel ein Reaktionsprodukt aus einem natürlichen oder hydrierten Rizinusöl und Ethylenoxid oder ein Polyoxyethylensorbitanfettsäureester ist.

11. Pharmazeutische Zusammensetzung nach einem der vorangehenden Ansprüche zur Verwendung bei der Behandlung eines Mehrfacharzneimittelresistenzsyndroms beim Menschen.

12. Verfahren zur Herstellung einer pharmazeutischen Zusammensetzung nach Anspruch 1, wobei das Verfahren das Zusammenmischen von Anteilen der hydrophilen Phase, des umgeesterten ethoxylierten Pflanzenöls und des oberflächenaktiven Mitteis und die Zugabe des Wirkstoffs umfaßt.

## Revendications

1. Composition pharmaceutique qui comprend de la ([3-désoxy-3'-oxo-MeBmt]¹-[Val]²-cyclosporine) comme ingrédient actif et un milieu support comprenant
1) une phase hydrophile ;
2) une huile végétale éthoxylée trans-estérifiée ; et
3) un agent tensioactif ;
cette composition étant sous la forme d'un préconcentré de micro-émulsion du type fournissant des micro-émulsions H/E (huile-dans-eau).

2. Composition pharmaceutique selon la revendication 1 dans laquelle l'huile végétale éthoxylée trans-estérifiée constitue de 10 à 85% en poids du milieu support ; l'agent tensioactif constitue de 5 à 80% en poids du milieu support ; et la phase hydrophile constitue de 10 à 50% en poids du milieu support.

3. Composition pharmaceutique selon la revendication 2 dans laquelle l'huile végétale éthoxylée trans-estérifiée constitue de 15 à 70% en poids du milieu support ; l'agent tensioactif constitue de 10 à 70% en poids du milieu support ; et la phase hydrophile constitue de 15 à 40% en poids du milieu support.

4. Composition pharmaceutique selon la revendication 3 dans laquelle l'huile végétale éthoxylée trans-estérifiée constitue environ 20% en poids du milieu support ; l'agent tensioactif constitue environ 60% en poids du milieu support ; et la phase hydrophile constitue environ 20% en poids du milieu support.

5. Composition pharmaceutique selon l'une quelconque des revendications précédentes dans laquelle la phase hydrophile inclut un composant choisi parmi le Transcutol, le Glycofurol et le 1,2-propylène glycol, ou leurs mélanges.

6. Composition pharmaceutique selon la revendication 5 dans laquelle la phase hydrophile inclut de l'éthanol comme autre co-composant hydrophile.

7. Composition pharmaceutique selon la revendication 5 dans laquelle l'éthanol constitue de 0 à 60% en poids de la phase hydrophile.

8. Composition pharmaceutique selon l'une quelconque des revendications précédentes dans laquelle l'ingrédient actif est présent en une quantité de 5 à 15% en poids de la composition.

9. Composition pharmaceutique selon l'une quelconque des revendications précédentes dans laquelle l'huile végétale éthoxylée trans-estérifiée est une huile de maïs éthoxylée trans-estérifiée.

10. Composition pharmaceutique selon l'une quelconque des revendications précédentes dans laquelle l'agent tensioactif est le produit de la réaction d'une huile de ricin naturelle ou hydrogénée et d'oxyde d'éthylène, ou est un ester d'acide gras de sorbitane polyoxyéthylé.

11. Composition pharmaceutique selon l'une quelconque des revendications précédentes pour utilisation dans le traitement du syndrome de résistance multiple aux médicaments chez l'homme.

12. Procédé de production d'une composition pharmaceutique telle que définie dans la revendication 1, dans lequel on mélange selon certaines proportions la phase hydrophile, l'huile végétale éthoxylée trans-estérifiée et l'agent tensioactif, et l'on ajoute l'ingrédient actif.
